**Europäisches Patentamt**

**European Patent Office**

**Office européen des brevets**

(11) Numéro de publication : **0 433 130 B1**

(12) **FASCICULE DE BREVET EUROPEEN**

(45) Date de publication du fascicule du brevet :
27.10.93 Bulletin 93/43

(51) Int. Cl.⁵ : **C07C 19/08, C07C 17/38**

(21) Numéro de dépôt : **90403439.4**

(22) Date de dépôt : **04.12.90**

(54) **Procédé pour éliminer le chlorure de vinylidène du 1,1-dichloro-1-fluoroéthane.**

(30) Priorité : **14.12.89 US 450654**

(43) Date de publication de la demande :
**19.06.91 Bulletin 91/25**

(45) Mention de la délivrance du brevet :
**27.10.93 Bulletin 93/43**

(84) Etats contractants désignés :
**BE DE ES FR GB GR IT NL**

(56) Documents cités :
**EP-A- 0 389 334**
**US-A- 2 894 044**
**US-A- 4 906 796**

(73) Titulaire : **ELF ATOCHEM S.A.**
**4 & 8, Cours Michelet La Défense 10**
**F-92800 Puteaux (FR)**

(72) Inventeur : **Yates, Stephen Frederic**
**2140 Goebbert Road No. 4-210**
**Arlington Heights, Illinois 60005 (US)**

(74) Mandataire : **Leboulenger, Jean et al**
**ATOCHEM Département Propriété Industrielle**
**Cédex 42- La Défense 10**
**F-92091 Paris la Défense (FR)**

## Description

Cette invention concerne la purification du 1,1-dichloro-1-fluoroéthane, désigné aussi par CFC-141b, qui présente un intérêt particulier comme remplaçant des chlorofluorocarbures ayant des propriétés physiques semblables, en particulier le CFC-11, le CFC-12 et le CFC-113. Le CFC-141b peut être préparé par réaction de chlorure de vinylidène ou de trichloroéthane avec HF. Ces procédés sont décrits, par exemple, dans les brevets U.S. 2 894 044 et 3 833 676.

Il est caractéristique de ces réactions qu'il se forme de nombreux sous-produits contenant des nombres différents d'atomes d'hydrogène, de chlore et de fluor sur les molécules de méthane, d'éthane et d'éthylène. Lorsque cela est possible, on peut séparer par distillation ces sous-produits et la matière première n'ayant pas réagi. D'autres composés sont relativement inoffensifs, car leur présence n'altère pas beaucoup les propriétés physiques pour lesquelles le CFC-141b est utile. Le chlorure de vinylidène a un point d'ébullition proche de celui du CFC-141b, ce qui les rend difficiles à séparer par distillation.

Il est souhaitable d'avoir une autre amélioration des procédés de purification du CFC-141b, notamment pour ce qui est de l'élimination du chlorure de vinylidène n'ayant pas réagi, et le présent inventeur a découvert un moyen de purification par adsorption qui va être décrit en détail ci-dessous.

RESUME DE L'INVENTION

Le chlorure de vinylidène peut être présent dans le CFC-141b en concentrations d'environ 200 à 900 ppm en poids, suivant le degré de conversion en CFC-141b et les étapes de séparation antérieures éventuelles. On peut éliminer par l'invention jusqu'à environ 95% du chlorure de vinylidène, en en laissant environ 50 à 200 ppm en poids dans le CFC-141b.

On fait passer le courant de CFC-141b sur un tamis moléculaire carboné ayant une grosseur moyenne de pore d'environ 4,2 à 4,5 angströms à une température de -20°C à 60°C et sous une pression de 100 à 500 kPa. Vis-à-vis de la plupart des autres impuretés que l'on s'attend à trouver dans le courant de CFC-141b, ces tamis moléculaire ont peu ou pas de capacité, ce qui rend l'élimination du chlorure de vinylidène très sélective.

Le procédé peut être réalisé avec du CFC-141b en phase liquide ou vapeur. Lorsqu'on utilise un lit fixe, on fait passer de la vapeur de CFC-141b sur les particules avec une vitesse spatiale horaire de gaz de 130 à 1500 h$^{-1}$. La vitesse spatiale de liquide correspondante pour une opération en phase liquide serait de 1 à 15 h$^{-1}$.

Brève description des dessins

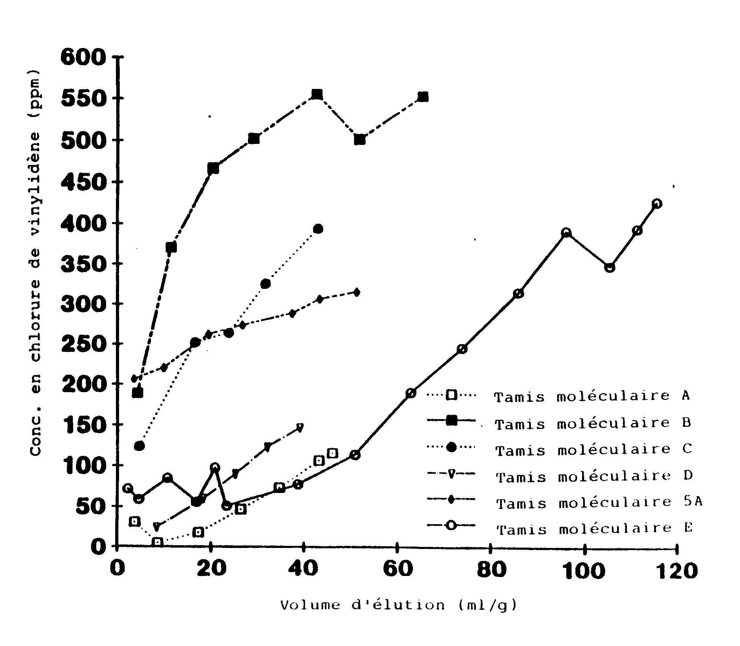

La seule figure est un graphique indiquant l'adsorption du chlorure de vinylidène sur divers tamis moléculaires carbonés.

Description des modes de réalisation préférés

Adsorption du chlorure de vinylidène

Le chlorure de vinylidène est présent dans le CFC-141b en quantités comprises entre environ 200 et 900 ppm en poids, suivant la conversion en CFC-141b et les étapes de purification préliminaires.

On préfère éliminer sélectivement le chlorure de vinylidène n'ayant pas réagi du CFC-141b. On utilise couramment une distillation, mais, lorsque les points d'ébullition sont proches, la séparation est difficile et coûteuse. Le chlorure de vinylidène et le CFC-141b entrent dans cette catégorie. On peut envisager l'adsorption pour ces séparations. Toutefois, il est possible qu'un adsorbant élimine plus que le composé cible, à savoir le chlorure de vinylidène, ce qui augmente son coût d'élimination. De plus, lorsque l'adsorbant sera régénéré, le composé cible sera contaminé par d'autres composés. Cependant, le présent inventeur a découvert qu'en choisissant l'adsorbant de type tamis moléculaire adéquat, on peut séparer le chlorure de vinylidène du CFC-141b et le recycler vers le procédé de fluoration.

Comme le montrent les exemples ci-dessous, le chlorure de vinylidène n'est pas éliminé dans une mesure significative par de nombreux tamis moléculaires, y compris la silicalite, CaX, CaY et la chabazite calcique (AW-500). Toutefois, les tamis moléculaires carbonés ayant une grosseur de pore d'environ 4,2 Å à 4,5 Å constituent un moyen efficace et sélectif de séparer le chlorure de vinylidène du CFC-141b.

2

Tamis moléculaires carbonés

Les tamis moléculaires carbonés sont disponibles dans le commerce. Ils sont habituellement dérivés de sources naturelles comme le charbon. Un exemple est les tamis moléculaires carbonés décrits dans un article de Juntgen et coll. de Bergbau-Forschung GmbH dans FUEL, 1981, vol. 60, septembre, p. 817-822.

Un autre tamis moléculaire carboné que l'on peut utiliser pour la purification des hydrocarbures fluorés est produit par un procédé particulier décrit dans le brevet U.S. 4 820 681 et cité ici à titre de référence. Ce procédé de fabrication peut être en gros caractérisé comme comprenant trois étapes: (1) polymérisation d'un monomère non oxygéné en présence d'un agent de réticulation non oxygéné; (2) formation de particules du polymère résultant en la forme souhaitée; et puis (3) carbonisation du matériau formé dans un environnement essentiellement non oxygéné.

Le monomère peut être choisi parmi un certain nombre de monomères différents. Ils doivent être facilement polymérisables, essentiellement non oxygénés dans leur structure moléculaire et de préférence constitués fondamentalement d'hydrogène, d'un halogène et de carbone. Parmi les substances que l'on peut employer comme monomère figurent l'acrylonitrile (AN), le fluorure de vinylidène (PVDF), le chlorotrifluoroéthylène (HALAR), le chlorure de vinylidène (PVCD), des mélanges de deux monomères ou plus, comme les mélanges de chlorure de vinylidène et de chlorure de vinyle, de chlorure de vinylidène et d'acrylonitrile, et un mélange de styrène et de divinylbenzène. D'autres monomères convenables sont le fluorure de vinyle, le bromure de vinyle, l'éthylène chloré, le chlorofluoroéthylène, le vinylchlorobenzène, le bromure de vinylidène et le fluorure de vinylidène-chlorotrifluoroéthylène. Le monomère préféré est le chlorure de vinylidène. Les réactions de polymérisation peuvent être réalisées selon un certain nombre de procédés différents connus dans la technique. Toutefois, on a obtenu les résultats les plus intéressants en employant une polymérisation en masse ou une polymérisation en solution.

Les polymères produits dans l'étape de polymérisation initiale doivent être réticulés avec un agent de réticulation essentiellement non oxygéné. L'agent de réticulation sera typiquement présent pendant la polymérisation en une concentration égale ou inférieure à 10% en moles du monomère. Un agent de réticulation préféré est le divinylbenzène. D'autres agents de réticulation possibles englobent le trivinylbenzène, le divinylacétylène et le sulfure de divinyle.

Comme on souhaite produire des tamis moléculaires carbonés à partir de polymères ne comportant pas de fonctions oxygénées, l'initiateur de polymérisation est aussi de préférence un composé non oxygéné. Par conséquent, on utilise de préférence un initiateur carboné ou azoïque plutôt qu'oxygéné.

La substance polymère est carbonisée par chauffage jusqu'à une température élevée dans un environnement essentiellement non oxygéné. Avant la carbonisation à haute température, la substance précurseur est soumise à une étape de chauffage ménagé au cours de laquelle sa température est élevée au-dessus de 150°C, par exemple à 240°C, et maintenue à cette valeur jusqu'à ce que l'on n'observe plus de perte de poids. La substance est ensuite de préférence soumise à une élévation programmée de température jusqu'à une température supérieure à 700°C, de préférence supérieure à 800°C, en particulier supérieure à 900°C. Les précurseurs de tamis dérivés de substances polymères sont essentiellement exempts des substances minérales comme les métaux et les oxydes minéraux qui peuvent être présents lorsque la substance précurseur est faite d'une substance naturelle comme le charbon, les écorces de noix de coco, la tourbe ou le bois. Les tamis préférés, sur une base non hydrogénée et non oxygénée, doivent contenir au moins 99,5% en poids de carbone et de préférence au moins 99,8% en poids de carbone.

Tandis que le procédé que l'on vient de décrire produit un tamis moléculaire carboné spécial et utile, on pense que sa grosseur moyenne de pore est légèrement supérieure à 3,8 angströms et, par conséquent, on doit encore le traiter pour accroître sa grosseur de pore afin qu'il satisfasse aux limites de grosseur de pore requises. On peut utiliser diverses techniques pour augmenter la grosseur des pores, comme un traitement avec de la vapeur d'eau à des températures comprises entre environ 700°C et 1000°C, un traitement avec de l'air à des températures comprises entre environ 400°C et 600°C, ou un traitement avec $CO_2$ à des températures comprises entre environ 700°C et 1000°C.

On notera qu'il est difficile de déterminer la grosseur de pore de tamis moléculaires carbonés et que, par conséquent, on ne dispose pas toujours de valeurs précises. Plusieurs approches ont été utilisées. Dans le premier procédé, on met en contact une série de molécules de taille croissante avec le tamis moléculaire carboné et on mesure la quantité adsorbée dans une balance de McBain. Lorsque la quantité d'une molécule adsorbée est sensiblement supérieure à celle trouvée avec d'autres molécules, on considère que la grosseur de pore a été déterminée. Dans le deuxième procédé, on étudie le comportement d'un mélange de gaz de taille moléculaire connue et de structures analogues en utilisant un tamis moléculaire carboné comme adsorbant chromatographique. On estime la grosseur des pores en observant lequel de ces gaz est retenu sur l'adsorbant. Un autre procédé encore nécessite la mesure de la chaleur isostérique d'adsorption d'un ou de plusieurs gaz.

La grosseur des pores est donnée par l'intersection d'une ligne tracée à cette énergie avec la courbe de potentiel de Lennard-Jones pour ce gaz. Un exemple de cette dernière technique est donné par K. Chihara et coll., dans le <u>Journal of Colloids and Interface Science</u>, <u>64</u>, 584 (1978), dans lequel on trouve que la grosseur des pores du tamis moléculaire MSC-5A est de 4,4 Å.

## Procédé

Lorsqu'on produit le CFC-141b par hydrofluoration catalytique du chlorure de vinylidène, la conversion en CFC-141b n'est que partielle et de nombreux sous-produits sont produits. Par conséquent, l'effluent du réacteur est séparé par distillation pour concentrer le produit CFC-141b et pour produire un courant de recyclage de la charge n'ayant pas réagi. Le courant de CFC-141b impur qui en résulte contient du HF et du chlorure de vinylidène n'ayant pas réagi, ainsi que des quantités mineures d'impuretés comme sous-produits. Le HF et le HCl peuvent être éliminés sélectivement par une technique décrite par d'autres et ne faisant pas partie de la présente invention. Une fois cela fait, le CFC-141b contient encore des impuretés qui doivent être éliminées, y compris environ 200 à 900 ppm en poids de chlorure de vinylidène. Le présent procédé a pour but d'éliminer le chlorure de vinylidène jusqu'à une proportion inférieure à 200 ppm en poids dans le CFC-141b, de préférence inférieure à 50 ppm en poids.

Le traitement du CFC-141b peut être réalisé en phase liquide ou en phase gazeuse, bien que l'on préfère la phase liquide pour éviter le coût de la vaporisation et de la condensation ultérieure. Diverses techniques connues de l'homme de métier peuvent être utilisées pour mettre en contact le courant de CFC-141b avec le tamis moléculaire carboné formant l'adsorbant, comme des lits fluides ou mobiles, mais, typiquement, on utilise un lit fixe de particules adsorbantes. Le choix de la granulométrie, de la forme du lit et de la vitesse spatiale du courant de CFC-141b est déterminé suivant les principes connus, selon les exigences pour obtenir l'élimination souhaitée du chlorure de vinylidène. Généralement, la vitesse spatiale horaire de gaz du courant de CFC-141b est de 130 à 1500 $h^{-1}$ lorsqu'on opère en phase gaz. La vitesse spatiale de liquide correspondante serait de 1 à 15 $h^{-1}$. L'adsorption est réalisée à une température convenable, généralement comprise entre -20°C et 60°C et sous une pression, qui dépend si l'on souhaite une mise en contact liquide ou vapeur, de 100 à 500 kPa.

Le lit adsorbant doit fournir une capacité optimale pour le chlorure de vinylidène, en faisant le bilan des coûts de l'installation et de l'adsorbant par rapport aux coûts de régénération. Lorsqu'on a atteint la capacité utile, on régénère l'adsorbant en chauffant le lit avec un courant gazeux pour éliminer le chlorure de vinylidène. Le CFC-141b restant dans le récipient et sur l'adsorbant est d'abord séparé et récupéré, puis le procédé de régénération est mis en oeuvre. Après avoir totalement chauffé le lit et éliminé le chlorure de vinylidène, on le refroidit et on le réintroduit dans le circuit. Les conditions nécessaires pour régénérer de façon optimale l'adsorbant seront déterminées en fonction de l'adsorbant utilisé et des utilités disponibles. Typiquement, on prévoit qu'un chauffage du lit d'adsorbant à 200°C-500°C avec un courant d'azote produira une régénération satisfaisante.

## Exemple 1

On étudie la capacité d'adsorption de chlorure de vinylidène d'un certain nombre d'adsorbants potentiels. On place dans une fiole de 20 ml, 1,0 g de l'adsorbant à étudier et un échantillon de 15 ml de CFC-141b impur contenant 576 ppm en poids de chlorure de vinylidène, 16 ppm en poids de dichloroacétylène, 840 ppm en poids de CFC-142b (1-chloro-1,1-difluoroéthane) et 20 ppm en poids de CFC-1131a (1-chloro-1-fluoroéthylène). Après une heure d'agitation, on prélève un échantillon du liquide et on l'analyse par chromatographie en phase gazeuse en utilisant deux colonnes en acier inoxydable en série de diamètre 3,175 mm (6,1 m de n-octane-Porasil C et 2 m de 0V-101 à 10% sur Chromosorb W, ces deux substances étant de 80/100 mesh, disponibles auprès de Alltech Associates) et 18 ml/min d'azote comme gaz vecteur. Les résultats figurent sur le tableau ci-dessous.

Tableau 1

| Adsorbant | Chlorure de vinylidène ppm en poids |
|---|---|
| Charge (sans adsorbant) | 576 |
| Chabazite (AW-500)[a] | 1340 |
| 5A[b] | 455 |
| 3A[c] | 608 |
| Calcium X[d] | 660 |
| Mordénite (AW-300)[e] | 1760 |
| Tamis mol. carboné[f] | 301 |
| Tamis mol. carboné[g] | 75 |
| Tamis mol. carboné[h] | 275 |
| Tamis mol. carboné[i] | 213 |

(a) fourni par UOP

(b) fourni par UOP

(c) fourni par UOP

(d) fourni par UOP

(e) fourni par UOP

(f) préparé par le procédé du brevet U.S. 4 820 681 au moyen de poly(chlorure de vinylidène) carbonisé à 800°C

(g) fourni par Takeda Chemical Co. (MSC-5A)

(h) fourni par Bergbau-Forschung GmbH

(i) fourni par Bergbau-Forschung GmbH, puis traité à la vapeur d'eau à 850°C pendant 30 minutes.

Le CFC-142b et le CFC-1131a ne sont pratiquement pas éliminés.

On constate que la plupart des adsorbants n'éliminent pas très bien le chlorure de vinylidène. En fait, la quantité de chlorure de vinylidène semble augmenter dans certains cas, ce qui est attribué à la déshydrofluoration du CFC-141b. Les tamis moléculaires carbonés adsorbent tous le chlorure de vinylidène et les différences de performances sont attribuées aux différences de grosseur de pore. On pense que le tamis moléculaire (f) a une grosseur de pore légèrement plus grande que 3,8 Å et il est moins efficace que le tamis moléculaire carboné (g) dont on a signalé une grosseur de pore de 4,4 Å. On pense que le tamis moléculaire (h) a une grosseur de pore intermédiaire et il donne des résultats intermédiaires, mais, lorsqu'il est traité à la vapeur d'eau pour ouvrir les pores (i), la capacité pour le chlorure de vinylidène augmente.

Exemple 2

En plus des expériences de sélection statiques décrites dans l'exemple 1, on réalise des essais dans lesquels on pompe un CFC-141b impur contenant 400 ppm en poids de chlorure de vinylidène, 840 ppm en poids de CFC-142b et 20 ppm en poids de CFC-1131a à 0,88 ml/min dans une colonne de diamètre 9,5 mm et de longueur 177,8 mm contenant 5-10 g de l'adsorbant à étudier (broyé à 20-50 mesh). Le débit est réglé en alimentant la charge à travers 6 mètres d'un tube capillaire en acier inoxydable de 0,0254 mm, la pression à la sortie étant maintenue à une valeur manométrique de 272 kPa. On prélève un échantillon du CFC-141b à la sortie de la colonne adsorbante au bout de 15-30 minutes et on l'analyse par chromatographie en phase gazeuse dans l'appareil décrit dans l'exemple 1.

## Tableau 2

| Adsorbant | CFC-142b (ppm) | CFC-1131a (ppm) | Chlorure de vinylidène (ppm) |
|---|---|---|---|
| Charge (sans adsorbant) | 840 | 20 | 400 |
| AW-500[a] | 870 | 20 | 680 |
| Silicalite[b] | 780 | 20 | 390 |
| Calcium X[c] | 830 | 30 | 420 |
| Calcium Y[d] | 900 | 20 | 380 |
| 5A[e] | 750 | 15 | 220 |
| Tamis mol. carboné[f] | 654 | 14 | 31 |
| Tamis mol. carboné[g] | 428* | 9 | 189 |

(a) Chabazite, fournie par UOP

(b) fourni par UOP

(c) fourni par UOP

(d) fourni par UOP

(e) fourni par UOP

(f) fourni par Takeda Chemical Co.

(g) préparé par le procédé du brevet U.S. 4 820 681 au moyen de poly(chlorure de vinylidène) carbonisé à 800°C

* Concentration dans la charge 453 ppm

On notera que la plupart des zéolithes ou bien ne présentent aucune affinité pour le chlorure de vinylidène ou bien conduisent à une nette hausse de la concentration en chlorure de vinylidène. On attribue les hausses, lorsqu'elles se présentent, à la décomposition du CFC-141b comme on l'a indiqué plus haut. Les meilleurs adsorbants sont les tamis moléculaires carbonés. Une comparaison des tamis moléculaires (f) et (g) montre l'importance de la dimension des pores. Le tamis (f) a une grosseur de pore de 4,4 Å, tandis que (g) a une grosseur de pore légèrement supérieure à 3,8 Å.

## Exemple 3

On étudie plusieurs tamis moléculaires carbonés en utilisant le mode opératoire décrit dans l'exemple 2, sauf que l'on analyse une série d'échantillons de CFC-141b pour déterminer la capacité des tamis moléculaires carbonés. Les résultats d'analyses pour le chlorure de vinylidène au cours de chaque essai sont indiqués sur la figure, qui donne les valeurs des teneurs en chlorure de vinylidène en fonction du volume de CFC-141b élué par gramme d'adsorbant utilisé. On calcule les capacités à partir de chaque courbe en notant le point auquel la courbe croise une ligne tracée à la moitié de la concentration initiale et en supposant que, jusqu'à ce point, tout le chlorure de vinylidène est adsorbé.

## Tableau 3

| Tamis moléculaire | Capacité (mg/g) | Source |
|---|---|---|
| A | 49,0 | Takeda Chemical Co. (HGR-805) |
| B | 3,34 | Préparé par le procédé du brevet U.S. 4 850 681 en utilisant du poly(chlorure de vinylidène) carbonisé à 800°C |
| C | 8,62 | Bergbau-Forschung |
| D | 28,0 | Préparé par traitement de carbone de Bergbau-Forschung à la vapeur d'eau à 850°C |
| E | 33,4 | Takeda Chemical Co. (MSC-5A) |

## Revendications

1. Procédé de purification de 1,1-dichloro-1-fluoroéthane (CFC-141b) contenant environ 200 à 900 ppm en poids de chlorure de vinylidène, comprenant le passage dudit 1,1-dichloro-1-fluoroéthane sur un tamis moléculaire carboné ayant une grosseur moyenne de pore comprise entre environ 4,2 et 4,5 angströms, à une température de -20°C à 60°C et sous une pression de 100 à 500 kPa et la récupération de 1,1-dichloro-1-fluoroéthane contenant moins de 200 ppm en poids de chlorure de vinylidène.

2. Procédé selon la revendication 1, dans lequel ledit tamis moléculaire carboné est un lit fixe de particules, le 1,1-dichloro-1-fluoroéthane est un gaz et la vitesse spatiale horaire de gaz dudit 1,1-dichloro-1-fluoroéthane est de 130 à 1500 h$^{-1}$.

3. Procédé selon la revendication 1, dans lequel ledit tamis moléculaire carboné est un lit fixe de particules, le 1,1-dichloro-1-fluoroéthane est un liquide et la vitesse spatiale horaire de liquide dudit 1,1-dichloro-1-fluoroéthane est de 1 à 15 h$^{-1}$.

4. Procédé selon la revendication 1, dans lequel le 1,1-dichloro-1-fluoroéthane récupéré contient moins de 50 ppm en poids de chlorure de vinylidène.

## Patentansprüche

1. Verfahren zur Reinigung von 1,1-Dichlor-1-fluorethan (CFC-141), das etwa 200 bis 900 ppm Vinylidenchlorid an Gewicht enthält. Das Verfahren beinhaltet das Durchleiten des genannten 1,1-Dichlor-1-fluorethans über ein kohlenstoffhaltiges Molekularsieb, das eine mittlere Porengröße zwischen etwa 4,2 und 4,5 Angström aufweist, bei einer Temperatur von -20°C bis 60°C und einem Druck von 100 bis 500 kPa, sowie die Rückgewinnung des 1,1-Dichlor-1-fluorethans, das nun weniger als 200 ppm Vinylidenchlorid an Gewicht enthält.

2. Verfahren nach Anspruch 1, in dem das genannte kohlenstoffhaltige Molekularsieb ein festes Teilchenbett ist, das 1,1-Dichlor-1-fluorethan ein Gas ist, und die stündliche Gasdurchgangsgeschwindigkeit des genannten 1,1-Dichlor-1-fluorethans 130 bis 1500 h$^{-1}$ beträgt.

3. Verfahren nach Anspruch 1, in dem das genannte kohlenstoffhaltige Molekularsieb ein festes Teilchenbett ist, das 1,1-Dichlor-1-fluorethan eine Flüssigkeit ist, und die stündliche Flüssigkeitsdurchgangsgeschwindigkeit des genannten 1,1-Dichlor-l-fluorethans 1 bis 15 h⁻¹ beträgt.

**4.** Verfahren nach Anspruch 1, in dem das rückgewonnene 1,1-Dichlor-1-fluorethan weniger als 50 ppm Vinylidenchlorid an Gewicht enthält.

**Claims**

1. Process for purification of 1,1-dichloro-1-fluoroethane (CFC-141b) containing approximately 200 to 900 ppm by weight of vinylidene chloride, comprising passing the said 1,1-dichloro-1-fluoroethane over a carbonaceous molecular sieve which has a mean pore size of between approximately 4.2 and 4.5 angstroms, at a temperature of -20°C to 60°C and at a pressure of 100 to 500 kPa and recovering 1,1-dichloro-1-fluoroethane containing less than 200 ppm by weight of vinylidene chloride.

2. Process according to Claim 1, in which the said carbonaceous molecular sieve is a stationary bed of particles, 1,1-dichloro-1-fluoroethane is a gas and the hourly gas space velocity of the said 1,1-dichloro-1-fluoroethane is from 130 to 1500 h⁻¹.

3. Process according to Claim 1, in which the said carbonaceous molecular sieve is a stationary bed of particles, 1,1-dichloro-1-fluoroethane is a liquid and the hourly liquid space velocity of the said 1,1-dichloro-1-fluoroethane is from 1 to 15 h⁻¹.

4. Process according to Claim 1, in which the recovered 1,1-dichloro-1-fluoroethane contains less than 50 ppm by weight of vinylidene chloride.

EP 0 433 130 B1